# FASCICULE DE BREVET EUROPEEN

(11) **EP 1 165 475 B1**
(45) Date de publication et mention de la délivrance du brevet: **14.01.2004**
(21) Numéro de dépôt: 00912607.9
(22) Date de dépôt: 15.03.2000
(51) Int. Cl.: C07C 17/38, C07C 19/08, C07C 17/20, C01B 7/19

(54) **PROCEDE DE SEPARATION DE FLUORURE D'HYDROGENE DE SES MELANGES AVEC DU 1,1,1,3,3-PENTAFLUOROBUTANE ET PROCEDE DE FABRICATION DE 1,1,1,3,3-PENTAFLUOROBUTANE**
VERFAHREN ZUR ABTRENNUNG VON FLUORWASSERSTOFF AUS SEINEN MISCHUNGEN MIT 1,1,1,3,3-PENTAFLUORBUTAN UND VERFAHREN ZUR HERSTELLUNG VON PENTAFLUORBUTAN
METHOD FOR SEPARATING HYDROGEN FLUORIDE FROM ITS MIXTURES WITH 1,1,1,3,3-PENTAFLUOROBUTANE AND METHOD FOR MAKING 1,1,1,3,3-PENTAFLUOROBUTANE

(30) Priorité: 24.03.1999 FR 9903917; 15.07.1999 FR 9909220
(43) Date de publication de la demande: 02.01.2002
(73) Titulaire: SOLVAY (Société Anonyme), 1050 Bruxelles (BE)
(72) Inventeur: LAMBERT, Alain, B-1320 Beauvechain (BE); WILMET, Vincent, B-1301 Wavre (BE)
(74) Mandataire: Jacques, Philippe
(86) Numéro de dépôt international: PCT/EP2000/002282
(87) Numéro de publication internationale: WO 2000/056687

(56) Documents cités:
- EP-A- 0 699 649
- EP-A- 0 905 085
- US-A- 5 208 398

## Description

La présente invention concerne un procédé de séparation de fluorure d'hydrogène de ses mélanges avec du 1,1,1,3,3-pentafluorobutane.

Le 1,1,1,3,3-pentafluorobutane (HFC-365mfc) peut être préparé par réaction d'un précurseur chloré approprié avec du fluorure d'hydrogène, tel que décrit par exemple dans la demande de brevet EP-A1-0699649 au nom de SOLVAY. Dans un tel procédé, à la sortie du réacteur d'hydrofluoration, le mélange de produits réactionnels contient, outre le 1,1,1,3,3-pentafluorobutane recherché, du chlorure d'hydrogène provenant de l'élimination du ou des atomes de chlore du précurseur chloré de départ, du fluorure d'hydrogène et, éventuellement, des diluants inertes ainsi que divers intermédiaires ou sous-produits en faibles quantités. Etant donné que l'on travaille habituellement avec un excès de fluorure d'hydrogène par rapport au précurseur chloré, il subsiste le plus souvent du fluorure d'hydrogène non converti dans le mélange de produits réactionnels. Alors que la plupart des constituants du mélange de produits réactionnels peuvent être facilement séparés complètement par distillation, une séparation complète entre le fluorure d'hydrogène et le 1,1,1,3,3-pentafluorabutane est très difficilement réalisable par distillation. On a en effet observé que ces composés forment un mélange azéotropique.

La présente invention a pour objet de fournir un procédé performant pour la séparation du fluorure d'hydrogène de ses mélanges avec du 1,1,1,3,3-pentafluorobutane.

A cet effet, l'invention concerne un procédé de séparation du fluorure d'hydrogène de ses mélanges avec du 1,1,1,3,3-pentafluorobutane, selon lequel la séparation est effectuée par extraction au moyen d'au moins un solvant organique contenant au moins un chloro(fluoro)butane de formule générale

CClₐF₃₋ₐCH₂CCl_{b}F_{2-b}CH₃ (I)

avec a un entier de 0 à 3, b un entier de 0 à 2, la somme de a et b étant au moins égale à 1.

De préférence, le solvant organique contient du 1,1,1,3,3-pentachlorobutane CCl₃-CH₂-CCl₂-CH₃, un chlorofluorobutane de formule CCl₂F-CH₂-CCl₂-CH₃, CClF₂-CH₂-CCl₂-CH₃, CF₃-CH₂-CCl₂-CH₃, CF₃-CH₂-CClF-CH₃, CCl₂F-CH₂-CClF-CH₃, CClF₂-CH₂-CClF-CH₃ et CClF₂-CH₂-CF₂-CH₃,CCl₃-CH₂-CClF-CH₃, CCl₃-CH₂-CF₂-CH₃, CCl₂F-CH₂-CF₂-CH₃, ou mélange de ces composés. De manière particulièrement préférée, le solvant organique contient du 1,1,1,3,3-pentachlorobutane.

Le solvant organique peut également contenir d'autres composés halogénés ou non-halogénés.

A titre d'exemple de composé non-halogéné pouvant éventuellement être présents dans le solvant organique, on peut citer les hydrocarbures contenant de 5 à 10 atomes de carbone, en particulier le n-pentane, le n-hexane, le n-heptane et le n-octane.

Des exemples de composés halogénés pouvant éventuellement être présents dans le solvant organique sont le chloroforme, le trichloréthylène, le tétrachloréthylène, le tétrachlorométhane, le 1,2-dichloréthane, le 1,1,1-trichloréthane, le 1,1,2-trichloréthane; le 1,1-dichloro-1-fluoroéthane (HCFC-141b), le 1,1,1- ou le 1,1,2-trifluorotrichloréthane, le 1,2,3-trichloropropane, des hydrocarbures perfluorés, le bromobenzène, l'o-dichlorobenzène, le p-chlorotoluène, le p-chlorotrifluorobenzène et le 1,2-dichloro-4-trifluorobenzène, de même que des mélanges de ces composés.

Le solvant organique est toutefois de préférence constitué essentiellement d'un ou de plusieurs chloro(fluoro)butanes de formule générale (1), le 1,1,1,3,3-pentachlorobutane constituant le solvant organique le plus préféré.

Parmi les solvants organiques contenant des chloro(fluoro)butanes de formule générale (I), ceux qui contiennent au moins un chlorofluorobutane contenant 3 ou 4 atomes de fluor sont préférés. Des exemples de chlorofluorobutanes de formule générale (I) contenant 3 ou 4 atomes de fluor, utilisables dans le solvant organique sont le 1,1-dichloro-1,3,3-trifluorobutane (HCFC-363kfc), le 3-chloro-1,1,1,3-tétrafluorobutane (HCFC-364mfb) et le 1-chloro-1,1,3,3-tétrafluorobutane (HCFC-3641fc). Ces composés peuvent être obtenus lorsqu'on soumet du 1,1,1,3,3-pentachlorobutane a une réaction d'hydrofluoration.

Dans une variante, le solvant organique contenant au moins un chloro(fluoro)butane de formule générale (I) contenant 3 ou 4 atomes de fluor contient en outre du 1,1,1,3,3-pentachlorobutane. Il peut être avantageux d'utiliser des mélanges contenant du 1,1,1,3,3-pentachlorobutane, afin d'améliorer les propriétés de décantation lors de l'extraction.

Une autre variante du procédé selon l'invention concerne un procédé dans lequel
(a) on soumet du 1,1,1,3,3-pentachlorobutane à une réaction d'hydrofluoration
(b) on soutire une quantité de mélange réactionel que l'on soumet à une opération de séparation pour récupérer au moins un solvant organique comprenant des chlorofluorobutanes de formule générale (1)
(c) on met en oeuvre le solvant organique dans le procédé de séparation selon l'invention.

L'opération de séparation est, par exemple, une distillation ou une démixtion à basse température. Les fractions autres que le solvant organique peuvent être recyclées vers la réaction d'hydrofluoration de 1,1,1,3,3-pentachlorobutane ou peuvent être soumises à des étapes de séparation ultérieures pour récupérer du 1,1,1,3,3-pentafluorobutane.

De manière préférée, on effectue l'extraction sur un mélange comprenant du HF et du HFC-365mfc dans des proportions proches de celles dans lesquelles ils forment une composition azéotropique. Le mélange peut en outre comprendre des quantités variables d'un chlorofluorobutane de formule générale (I). Souvent le chlorofluorobutane est un chlorofluorobutane de formule générale (I) dans laquelle la somme de a et b est égale à1 ou 2.

Si le mélange initial de fluorure d'hydrogène et de HFC-365mfc s'écarte de la composition azéotropique, il peut être avantageux d'effectuer au préalable une distillation pour séparer l'azéotrope du composé en excès. La composition azéotropique est ensuite soumise à l'extraction.

Dans le procédé de séparation selon l'invention, le rapport pondéral entre le solvant organique et le mélange de fluorure d'hydrogène et de 1,1,1,3,3-pentafluorobutane est généralement d'au moins 0. 1. De préférence, on travaille avec un rapport pondéral d'au moins 0.2. Le rapport pondéral entre le solvant organique et le mélange de fluorure d'hydrogène et de 1,1,1,3,3-pentafluorobutane ne dépasse en général pas 10. De préférence, il ne dépasse pas 5.

La température à laquelle l'extraction est effectuée est généralement d'au moins - 25°C. De préférence, elle est d'environ -10°C. La température ne dépasse en général pas 40°C. De préférence, elle ne dépasse pas 30°C.

Le procédé selon l'invention est réalisé à une pression suffisante pour maintenir le mélange à l'état liquide. Il peut être réalisé sous la pression autogène du mélange; dans ce cas la pression est généralement inférieure à 3 bar. Alternativement, il peut être réalisé à une pression supérieure à la pression autogène. Dans ce cas, la pression totale sera généralement inférieure à 10 bar; de manière préférée, la pression mise en oeuvre sera inférieure à 3 bar mais supérieure à 1 bar.

La mise en contact du mélange de fluorure d'hydrogène et de 1,1,1,3,3-pentafluorobutane avec le solvant organique d'extraction est réalisée en une ou plusieurs étapes, au moyen de n'importe quel dispositif conventionnel d'extraction liquide-liquide, par exemple par mise en contact intime au moyen d'un mélangeur statique, d'un réacteur agité, d'un extracteur à disques rotatifs, d'un extracteur à centrifugation ou d'une colonne à plateaux perforés, fonctionnant soit à contre-courant, soit à co-courant. De préférence, la mise en contact est effectuée au sein d'un réacteur agité. L'extraction peut être menée en continu ou en discontinu. De préférence, elle est menée en continu.

Après extraction, on sépare une phase organique enrichie en 1,1,1,3,3-pentafluorobutane d'une phase enrichie en fluorure d'hydrogène (appelée ci-après phase HF). Cette séparation peut être réalisée simplement par décantation, mais on peut aussi mettre en oeuvre tout autre dispositif classique de séparation de phases, tel qu'une centrifugation ou une séparation par hydrocyclone. La séparation par.décantation est préférée.

La phase organique comprend principalement le solvant d'extraction enrichi en 1,1,1,3,3-pentafluorobutane, mais peut aussi contenir une certaine quantité de fluorure d'hydrogène. Sa composition correspond le plus souvent à la composition d'équilibre, déterminée par les coefficients de partage des différents composés entre le fluorure d'hydrogène et le solvant d'extraction.

Du 1,1,1,3,3-pentafluorobutane peut aisément être séparé des autres constituants de la phase organique par une technique de séparation classique telle qu'une distillation. Le 1,1,1,3,3-pentafluorobutane peut ensuite être traité par voie humide pour éliminer les dernières traces d'acidité et/ou adsorbé sur charbon actif et/ou désacidifié sur zéolithe ou alumine. Le solvant peut être recyclé partiellement ou totalement à l'étape d'extraction.

Lorsque le solvant d'extraction est essentiellement constitué de chloro(fluoro)butanes de la formule générale (I), il peut, après séparation du 1,1,1,3,3-pentafluorobutane extrait, être envoyé tel quel, partiellement ou totalement, directement à un réacteur de fabrication de 1,1,1,3,3-pentafluorobutane par hydrofluoration de tels chloro(fluoro)butanes de la formule générale (I), en particulier par hydrofluoration de 1,1,1,3,3-pentachlorobutane.

La phase HF contient principalement du fluorure d'hydrogène appauvri en 1,1,1,3,3-pentafluorobutane, mais peut aussi contenir une quantité non négligeable de solvant d'extraction.

Lorsque le solvant d'extraction est essentiellement constitué de chloro(fluoro)butanes de la formule générale (I), la phase HF peut être envoyée telle quelle directement à un réacteur de fabrication de 1,1,1,3,3-pentafluorobutane par hydrofluoration de tels chloro(fluoro)butanes de la formule générale (I), en particulier par hydrofluoration de 1,1,1,3,3-pentachlorobutane. Avec des solvants contenant un composé qui n'est pas un précurseur du 1,1,1,3,3-pentafluorobutane, une telle solution n'est possible que lorsque ce composé est suffisamment inerte dans les conditions réactionnelles.

L'invention concerne aussi un procédé pour la fabrication de 1,1,1,3,3-pentafluorobutane par hydrofluoration de chloro(fluoro)butanes de la formule générale (I), dans lequel on introduit dans un réacteur de fabrication de 1,1,1,3,3-pentafluorobutane des chloro(fluoro)butanes de la formule générale (I) ayant servi comme solvant d'extraction dans le procédé de séparation selon l'invention. Dans un tel procédé de fabrication, on effectue au moins partiellement l'alimentation du réacteur en produit(s) de départ par du solvant d'extraction usagé.

Dans une première variante du procédé de fabrication selon l'invention, du solvant d'extraction usagé est introduit dans le réacteur d'hydrofluoration sous la forme de la phase HF obtenue dans le procédé de séparation selon l'invention.

Dans une deuxième variante du procédé de fabrication selon l'invention, éventuellement cumulée à la première variante ci-dessus, du solvant d'extraction usagé est introduit dans le réacteur d'hydrofluoration sous la forme de la phase organique obtenue dans le procédé de séparation selon l'invention, de laquelle on a essentiellement séparé le HFC-365mfc.

De préférence le solvant usagé alimentant le réacteur d'hydrofluoration comprend du 1,1,1,3,3-pentachlorobutane, avantageusement en une teneur d'au moins 50% en poids. De préférence la teneur en 1,1,1,3,3-pentachlorobutane dans le solvant usagé est d'au moins 80% en poids.

Dans le procédé de fabrication de 1, 1, 1,3,3-pentafluorobutane selon l'invention, on peut utiliser des techniques d'hydrofluoration connues, en présence ou en absence d'un catalysateur d'hydrofluoration.

Les exemples qui suivent sont destinés à illustrer la présente invention sans toutefois en limiter la portée.

### Exemples 1 à 6

Dans un autoclave de 0.5 1 en INOX 316 équipé d'un agitateur à pales, de deux tubes plongeants permettant de prélever des échantillons des deux phases liquides (au fond et en haut du réacteur) et d'un doigt de gant muni d'un thermocouple permettant de mesurer la température, on a introduit un mélange de 1,1,1,3,3-pentafluorobutane (HFC-365mfc) et de HF proche de la composition azéotropique et on l'a extrait au moyen d'un solvant. Le solvant utilisé ainsi que les quantités en poids de HFC-365mfc, de HF et de solvant mis en oeuvre sont repris dans le tableau ci-après. L'autoclave est plongé dans un bain maintenu à température constante. L'extraction a été effectuée à -10°C sous une pression autogène c'est-à-dire légèrement inférieure à 1 bar. Le mélange a été agité pendant 1 heure (exemple 3, 5 et 6), 4 heures (ex. 1 et 2) ou 24 heures (ex. 4), puis on l'a laissé décanter pendant au moins 1 heure.

Pour chaque exemple, l'efficacité de l'extraction a été évaluée par analyse de prélèvements effectués dans chacune des deux phases liquides. Ces prélèvements de liquide ont été réalisés via un sas de 5 cm3 environ après avoir pressurisé l'autoclave sous 2 bar d'azote.

Les résultats des analyses sont repris dans le tableau ci-après.

**TABLEAU**

| Ex | Solvant | Composition de départ (g) | | | HFC/HF au départ (g/g) | Composition de la phase organique (% en poids) | | | HFC/HF Dans la phase Organique (g/g) |
|---|---|---|---|---|---|---|---|---|---|
| | | HFC | HF | Solv. | | HFC | HF | Solv. | |
| 1 | Tétrachloroéthylène | 130 | 93 | 102 | 1,4 | 11,8 | - | 88,2 | - |
| 2 | Tétrachloroéthylène | 101 | 93 | 100 | 1,1 | 9,3 | 0,1 | 90,6 | 93,0 |
| 3 | 1,1,1,3,3-pentachlorobutane | 102 | 106 | 106 | 0,96 | 18,8 | 0,13 | 81,1 | 144,6 |
| 4 | 1,1,1,3,3-pentachlorobutane | 151 | 139 | 42 | 1,08 | 30,1 | 0,5 | 69,3 | 60,2 |
| 5 | 1,1-dichloro-1,3,3-trifluorobutane* | 116 | 113 | 109 | 1,03 | 45,3 | 2,5 | 52,1 | 18,1 |
| 6 | Chlorotétrafluorobutanes/ PCBa** | 102 | 119 | 98 | 0,86 | 39,8 | 1,2 | 59,0 | 33,2 |

| | | | | | | | | | |
|---|---|---|---|---|---|---|---|---|---|
| * produit technique constitué d'un mélange de produits en C4, avec une teneur en HCFC-363kfc de 95% en poids. | | | | | | | | | |
| ** produit technique contenant un mélange de produits en C4 dont 80 % en poids de HCFC-364mfb et 12,5 % en poids de HCFC-3641fc. Le rapport pondéral entre les chlorotétrafluorobutanes et le 1, 1, 1,3,3-pentachlorobutane (PCBa) dans le solvant organique est de 0,88. | | | | | | | | | |

Les résultats du tableau montrent que la concentration en 1,1,1,3,3-pentafluorobutane (HFC-365mfc) dans la phase organique est d'environ 9 à 11,% en poids lorsque du tétrachloroéthylène est utilisé comme solvant d'extraction et qu'elle est d'environ 18 à 30% en poids lorsque du 1,1,1,3,3-pentachlorobutane est utilisé comme solvant d'extraction. Avec le mélange de chlorotétrafluorobutanes et de 1,1,1,3,3-pentachlorobutane la teneur en 1,1,1,3,3-pentafluorobutane, dans la phase organique a atteint environ 40%. Avec le 1,1-dichloro-1,3,3-trifluorobutane on a obtenu une teneur en 1,1,1,3,3-pentafluorobutane dans la phase organique d'environ 45%.

Il apparaît donc que le 1,1,1,3,3-pentachlorobutane extrait à peu prés deux fois plus de HFC-365mfc que le tétrachloroéthylène tout en conservant un rapport élevé HFC-365mfc/HF dans la phase organique. De plus, la densité élevée du 1,1,1,3,3-pentachlorobutane assure de bonnes propriétés quant à la décantation des phases lors de l'étape d'extraction. En outre, le 1,1,1,3,3-pentachlorobutane ne forme pas d'azéotrope avec le HFC-365mfc qui peut dès lors être séparé de mélanges avec le 1,1,1,3,3-pentachlorobutane facilement par distillation.

La mise en oeuvre de composés chlorofluorobutanes contenant 3 ou 4 atomes de fluor (HCFC-363kfc, HCFC-364-mfb, HCFC-364-lfc) purs ou en mélanges permet d'augmenter encore l'efficacité d'extraction du 1,1,1,3,3-pentafluorobutane dans la phase organique. On arrive à des taux d'extraction du 1,1,1,3,3-pentafluorobutane allant d'environ 55% jusqu'à environ 71%. Le taux d'extraction indique le pourcentage de 1,1,1,3,3 -pentafluorobutane extrait dans la phase organique par rapport à la quantité totale de 1,1,1,3,3-pentafluorobutane dans la composition de départ.

La phase organique débarrassée du 1,1,1,3,3-pentafluorobutane et la phase HF peuvent être recyclées vers un réacteur de fabrication de 1,1,1,3,3-pentafluorabutane.

## Revendications

1. Procédé de séparation de fluorure d'hydrogène de ses mélanges avec du 1,1,1,3,3-pentafluorobutane, selon lequel la séparation est effectuée par extraction au moyen d'un solvant organique contenant au moins un chloro(fluoro)butane de formule générale CClaF3-aCH2CClbF2-bCH3, avec a un entier de 0 à 3, b un entier,de 0 à 2, la somme de a et b étant au moins égale à 1.

2. Procédé selon la revendication 1, dans lequel le solvant organique contient du 1,1,1,3,3-pentachlorobutane (a=3 et b=2).

3. Procédé selon la revendication 2, dans lequel le solvant organique est essentiellement constitué de 1,1,1,3,3-pentachlorobutane.

4. Procédé selon la revendication 1, dans lequel le chloro(fluoro)butane contient 3 ou 4 atomes de fluor.

5. Procédé selon la revendication 4, dans lequel le solvant organique contient en outre du 1,1,1,3,3-pentachlorobutane

6. Procédé selon l'une quelconque des revendications 1 à 5, dans lequel le rapport pondéral entre le solvant organique et le mélange de fluorure d'hydrogène et d'hydrofluoroalcane est compris entre 0.1 et 10.

7. Procédé selon l'une quelconque des revendications 1 à 6, dans lequel l'extraction est effectuée à une température comprise entre -25°C et 40°C.

8. Procédé selon l'une quelconque des revendications 1 à 7, dans lequel l'extraction est effectuée sous la pression autogène du mélange.

9. Procédé selon l'une quelconque des revendications 1 à 8 dans lequel l'extraction est effectuée à une pression totale inférieure à 10 bar.

10. Procédé selon l'une quelconque des revendications 1 à 9, dans lequel l'extraction est suivie par au moins une distillation de la phase organique et/ou de la phase HF.

11. Procédé selon l'une quelconque des revendications 1 à 10 dans lequel
(a) on soumet du 1,1,1,3,3-pentachlorobutane à une réaction d'hydrofluoration
(b) on soutire une quantité de mélange réactionel que l'on soumet à une opération de séparation pour récupérer au moins un solvant organique comprenant des chlorofluorobutanes de formule générale (I)
(c) on met en oeuvre le solvant organique dans le procédé de séparation selon l'une quelconque des revendications 1 à 10.

12. Procédé de fabrication de 1, 1, 1,3,3 -pentafluorobutane dans lequel
(a) on prépare du 1,1,1,3,3-pentafluorobutane par réaction d'un précurseur chloré approprié avec du fluorure d'hydrogène, de manière à obtenir un mélange de produits réactionnels contenant du 1,1,1,3,3-pentafluorobutane et du fluorure d'hydrogène non converti;
(b) on soumet le mélange de produits réactionnels, éventuellement après distillation, au procédé de séparation selon l'une quelconque des revendications 1 à 11, de manière à obtenir une phase organique enrichie en 1,1,1,3,3-pentafluorobutane et une phase enrichie en HF;
(c) on sépare du 1,1,1,3,3-pentafluorobutane des autres constituants de la phase organique obtenue à l'étape (b).

13. Procédé selon la revendication 12, dans lequel le précurseurs chloré mis en oeuvre à l'étape (a) est constitué au moins partiellement de chloro(fluoro)butanes de formule CClaF3-aCH2CClbF2-bCH3, avec a un entier de 0 à 3, b un entier de 0 à 2, la somme de a et b étant au moins égale à 1, ayant servi comme solvant d'extraction à l'étape (b) dans un réacteur de fabrication de 1,1,1,3,3-pentafluorobutane par hydrofluoration desdits chloro(fluoro)butanes.

## Patentansprüche

1. Verfahren zur Abtrennung von Fluorwasserstoff aus seinen Gemischen mit 1,1,1,3,3-Pentafluorbutan, wonach die Abtrennung durch Extraktion mit einem organischen Lösungsmittel vorgenommen wird, das wenigstens ein Chlor(fluor)butan der allgemeinen Formel CClₐF₃₋ₐCH₂CCl_{b}F_{2-b}CH₃, enthält, worin a eine ganze Zahl von 0 bis 3 ist, b eine ganze Zahl von 0 bis 2 ist und die, Summe aus a und b wenigstens den Wert 1 aufweist.

2. Verfahren nach Anspruch 1, worin das organische Lösungsmittel 1,1,1,3,3-Pentachlorbutan (a=3, b=2) enthält.

3. Verfahren nach Anspruch 2, worin das organische Lösungsmittel im wesentlichen aus 1, 1, 1,3,3-Pentachlorbutan besteht.

4. Verfahren nach Anspruch 1, worin das Chlor(fluor)butan 3 oder 4 Fluoratome enthält.

5. Verfahren nach Anspruch 4, worin das organische Lösungsmittel weiterhin 1,1,1,3,3-Pentachlorbutan enthält.

6. Verfahren nach einem der Ansprüche 1 bis 5, worin das Gewichtsverhältnis zwischen dem organischen Lösungsmittel und dem Gemisch aus Fluorwasserstoffund Hydrofluoralkan zwischen 0,1 und 10 beträgt.

7. Verfahren nach einem der Ansprüche 1 bis 6, worin die Extraktion bei einer Temperatur zwischen -25°C und 40°C ausgeführt wird.

8. Verfahren nach einem der Ansprüche 1 bis 7, worin die Extraktion unter dem autogenen Druck des Gemisches ausgeführt wird.

9. Verfahren nach einem der Ansprüche 1 bis 8, worin die Extraktion bei einem Gesamtdruck von unter 10 bar ausgeführt wird.

10. Verfahren nach einem der Ansprüche 1 bis 9, worin auf die Extraktion wenigstens eine Destillation der organischen Phase und/oder der HF-Phase folgt.

11. Verfahren nach einem der Ansprüche 1 bis 10, worin
(a) 1,1,1,3,3-Pentachlorbutan einer Hydrofluorierungsreaktion unterzogen wird,
(b) eine Menge an Reaktionsgemisch abgezogen wird, die einer Trennbehandlung unterworfen wird, um wenigstens ein organisches Lösungsmittel zu gewinnen, das Chlorfluorbutane der allgemeinen Formel (I) umfaßt,
(c) das organische Lösungsmittel in dem Trennverfahren gemäß einem der Ansprüche 1 bis 10 eingesetzt wird.

12. Verfahren zur Herstellung von 1, 1, 1,3,3-Pentafluorbutan, worin
(a) 1, 1, 1, 3,3 -Pentafluorbutan durch Reaktion eines entsprechenden chlorierten Vorläufers mit Fluorwasserstoff hergestellt wird, um ein Gemisch von Reaktionsprodukten zu erhalten, das 1,1,1,3,3-Pentafluorbutan und nicht umgewandelten Fluorwasserstoff enthält;
(b) das Gemisch der Reaktionsprodukte, gegebenenfalls nach Destillation, dem Trennverfahren nach einem der Ansprüche 1 bis 11 unterworfen wird, um eine an 1,1,1,3,3,-Pentafluorbutan angereicherte organische Phase und eine an HF-angereicherte Phase zu erhalten;
(c) das 1,1,1,3,3-Pentafluorbutan von anderen Bestandteilen der in Stufe (b) erhaltenen organischen Phase abgetrennt wird.

13. Verfahren nach Anspruch 12, worin der in Stufe (a) eingesetzte chlorierte Vorläufer wenigstens zum Teil aus Chlor(fluor)butanen der Formel CClₐF₃₋ₐ CH₂CCl_{b}F_{2-b}CH₃ besteht, worin a eine ganze Zahl von 0 bis 3 ist, b eine ganze Zahl von 0 bis 2 ist und die Summe aus a und b wenigstens einen Wert von 1 aufweist, die in Stufe (b) als Extraktionslösungsmittel in einem Reaktor zur Herstellung von 1, 1 , 1,3,3-Pentafluorbutan durch Hydrofluorierung der Chlor(fluor)butane gedient haben.

## Claims

1. Process for the separation of hydrogen fluoride from its mixtures with 1,1,1,3,3-pentafluorobutane, according to which the separation is carried out by extraction using an organic solvent containing at least one chloro(fluoro)butane of general formula CClₐF₃₋ₐCH₂CCl_{b}F_{2-b}CH₃, with a an integer from 0 to 3, b an integer from 0 to 2, the sum of a and b being at least equal to 1.

2. Process according to Claim 1, in which the organic solvent contains 1, 1, 1,3,3-pentachlorobutane (a=3 and b=2).

3. Process according to Claim 2, in which the organic solvent essentially consists of 1,1,1,3,3-pentachlorobutane.

4. Process according to Claim 1, in which the chloro(fluoro)butane contains 3 or 4 fluorine atoms.

5. Process according to Claim 4, in which the organic solvent contains, in addition, 1, 1, 1,3,3 -pentachlorobutane.

6. Process according to any one of Claims 1 to 5, in which the weight ratio between the organic solvent and the mixture of hydrogen fluoride and hydrofluoroalkane is between 0.1 and 10.

7. Process according to any one of Claims 1 to 6, in which the extraction is carried out at a temperature of between -25°C and 40°C.

8. Process according to any one of Claims 1 to 7, in which the extraction is carried out at the autogenous pressure of the mixture.

9. Process according to any one of Claims 1 to 8, in which the extraction is carried out at a total pressure of less than 10 bar.

10. Process according to any one of Claims 1 to 9, in which the extraction is followed by at least one distillation of the organic phase and/or of the HF phase.

11. Process according to any one of Claims 1 to 10, in which
(a) 1,1,1,3,3-pentachlorobutane is subjected to a hydrofluorination reaction
(b) a quantity of reaction mixture is drawn off and subjected to a separation operation in order to recover at least one organic solvent comprising chlorofluorobutanes of general formula (I)
(c) the organic solvent is used in the separation process according to any one of Claims 1 to 10.

12. Process for the manufacture of 1,1,1,3,3-pentafluorobutane in which:
(a) 1,1,1,3,3- pentafluorobutane is prepared by reaction of an appropriate chlorinated precursor to obtain a mixture of reaction products containing 1,1,1,3,3- pentafluorobutane and non-converted hydrogen fluoride;
(b) the mixture of reaction products is subjected, optionally after distillation, to the separation process according to anyone of claims 1 to 11, to obtain an organic phase enriched in 1,1,1,3,3-pentafluorobutane and a phase enriched in HF;
(c) 1,1,1,3,3- pentafluorobutane is separated from the other constituents of the organic phase obtained in step (b).

13. Process according to claim 12 in which the chlorinated precursor used in step (a) consists at least partially of chloro(fluoro)butanes of general formula CClₐF₃₋ₐCH₂CCl_{b}F_{2-b}CH₃, with a an integer from 0 to 3, b an integer from 0 to 2, the sum of a and b being at least equal to 1, which have been used as extraction solvent in step (b) in a reactor for the manufacture of 1,1,1,3,3-pentafluorobutane by hydrofluorination of the said chloro(fluoro)butanes.
